**Europäisches Patentamt**

**European Patent Office**

**Office européen des brevets**

(19)

(11) Veröffentlichungsnummer : **0 051 082**
**B1**

(12)

# EUROPÄISCHE PATENTSCHRIFT

(45) Veröffentlichungstag der Patentschrift :
**10.02.88**

(21) Anmeldenummer : **80106734.9**

(22) Anmeldetag : **03.11.80**

(51) Int. Cl.⁴ : **A 01 C   3/02**, **C 12 M   1/00**,
**C 02 F   3/28**

(54) **Fermenter für Biogasanlagen.**

(43) Veröffentlichungstag der Anmeldung :
**12.05.82 Patentblatt 82/19**

(45) Bekanntmachung des Hinweises auf die Patenterteilung : **10.02.88 Patentblatt 88/06**

(84) Benannte Vertragsstaaten :
**AT BE CH DE FR GB IT LI LU NL SE**

(56) Entgegenhaltungen :
**DE-C-   852 378**
**DE-C-   867 530**
**MECHANICAL ENGINEERING, Band 101, Nr. 11,**
**November 1979 New York R.C. McDONALD et al.**
**"Fuel Gas from Biodigestion" Seite 62**

(73) Patentinhaber : **Buchner, Sigmund J.**
**Hofdorf - Müllergasse 5**
**D-8404 Wörth (DE)**

**Strasser, Albert**
**Lettnerstrasse 16**
**D-8068 Pfaffenhofen a.d. Ilm (DE)**

**Müller, Klaus Maximilian**
**Plinganserstrasse 32 / Gartenhaus**
**D-8000 München 70 (DE)**

(72) Erfinder : **Neubauer, Josef**
**Wehrlestrasse 4**
**D-8000 München 80 (DE)**
Erfinder : **Buchner, Sigmund J.**
**Hofdorf-Müllergasse 5**
**D-8404 Wörth an der Ilm (DE)**
Erfinder : **Strasser, Albert**
**Lettnerstrasse 16**
**D-8068 Pfaffenhofen an der Ilm (DE)**

(74) Vertreter : **Siebert . Grättinger & Bockhorni**
**Postfach 1649 Almeidaweg 35**
**D-8130 Starnberg (München) (DE)**

## Beschreibung

Die Erfindung bezieht sich auf einen um seine Längsachse drehbar gelagerten zylinderförmigen Fermenter für Biogasanlagen.

Bekannte Fermenter dieser Bauart (Mechanical Engineering, Bd. 101, Nr. 11, Nov. 79, S. 62) erfordern eine aufwendige Lagerung, wenn sie im kontinuierlichen Betrieb mit ständig wechselnden füllmengen arbeiten. Auch die Speicherung der Prozesswärme bereitet Schwierigkeiten bzw. es sind verhältnismäßig hohe Wärmeverluste in Kauf zu nehmen.

Allgemein wird mit derartigen Biogasanlagen durch Befüllung mit niederwertigen Naturprodukten (Biomasse) — tierischen, wie pflanzlichen Abfällen und Reststoffen in flüssiger Phase, sowie organischen Stoffen verschiedener Arten in fester Phase — Methangas erzeugt, wobei die anerobe Ausfaulung durch Einhaltung einer bestimmten, möglichst konstanten Temperatur gefördert wird.

In diesem Zusammenhang ist bekannt, daß durch den Betrieb bei Prozesstemperaturen um 50 °C und darüber die Methangasausbeute deutlich gesteigert werden kann. Andererseits fehlen technische Lösungen, die einen Betrieb unter derartigen Bedingungen rentabel machen. Dabei ist neben dem Energiebedarf zum Aufheizen des frischen Substrates besonders der Energieaufwand für die Aufrechterhaltung der Prozesstemperatur im laufenden Betrieb zu beachten.

Somit liegt der vorliegenden Erfindung die Aufgabe zugrunde, diesen hohen Einsatz an Prozessenergie zu reduzieren, wobei nach einer Lösung gesucht wird, bei welcher die mechanische Durchmischung des Substrates uneingeschränkt erhalten bleibt.

Diese Aufgabe wird dadurch gelöst, daß der Fermenter in Wasser oder einer anderen Flüssigkeit derart schwimmend gelagert ist, daß sein Eigengewicht mit der Befüllung durch den Auftrieb weitgehend kompensiert ist.

Der rotierende, zylinderförmige Fermenter liegt in einem Wärmebad, dessen Wärmeverluste durch eine gute Isolierung gering gehalten werden können. Zu diesem Zweck ist der Fermenter bevorzugt in einem Wasserdurchlauftank angeordnet, der als schwimmendes Lager und als konstanter Prozesswärmespender für den Fermenter ausgebildet ist.

Die schwimmende Lagerung des Fermenters ist je nach Anlagengröße so zu dimensionieren, daß das Eigengewicht des Fermenters mit Befüllung weitgehend durch die Auftriebskräfte aufgehoben wird. Unter dieser Voraussetzung ist es möglich, den Fermenter·durch Mittel in Rotation zu versetzen, die durch den im Fermenter sich aufbauenden Gasdruck angetrieben sind.

Der Gärprozess wird noch wesentlich gefördert durch im Inneren des Fermenters angeordnete Mischarme, die eine Durchmischung der Befüllung während der Rotation des Fermenters bewirken.

Die hier verwirklichte Lösung ist mit dem Vormagensystem der Kuh vergleichbar, deren Pansen einem Gärbehälter entspricht.

Der vorliegende Lösungsvorschlag ermöglicht die Aufrechterhaltung einer hohen Prozesswärme bei reduzierten Wärmeverlusten. Der durch die Drehung des Fermenters erzielbare gute Rühreffekt, durch welchen eine unerwünschte Schwimmdeckenbildung weitgehend vermeidbar ist, wird nicht durch erhöhte Wärmeverluste erkauft. Die vorgeschlagene Lagerung des Fermenters im Wärmebad ermöglicht bei guter Isolierung u. energiearmen Betrieb des Fermenters eine kostengünstige Bauweise durch industrielle Fertigung.

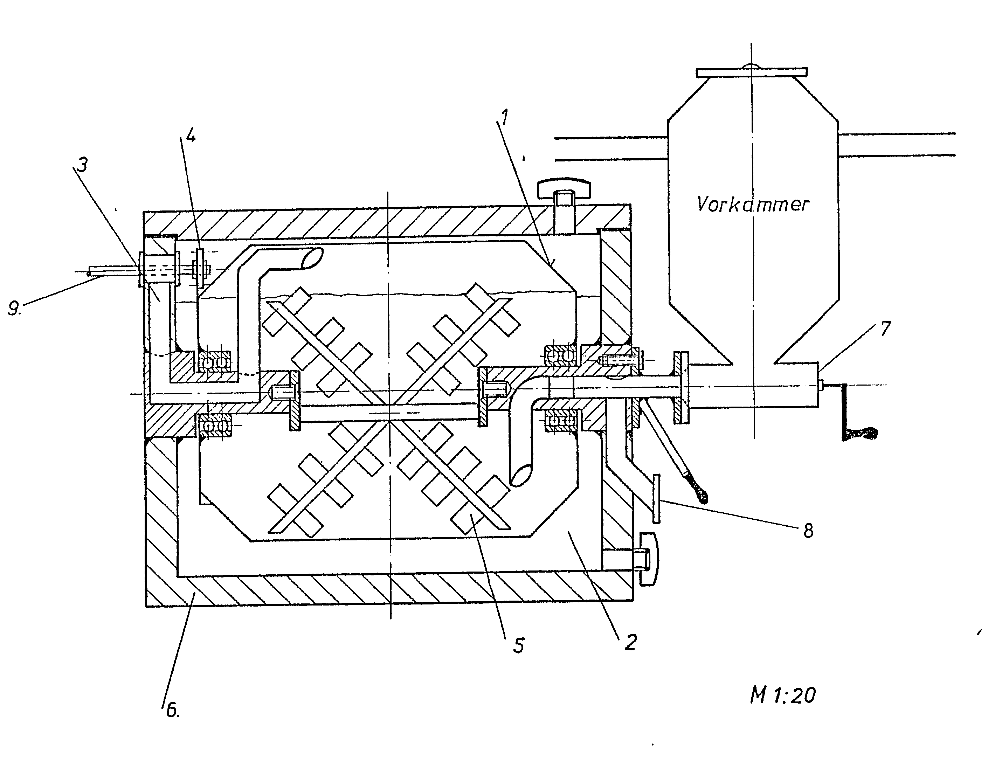

Im folgenden wird ein Ausführungsbeispiel der Erfindung anhand der Zeichnung erläutert.

Die Anlage ist als Wasserdurchlauftank 6 ausgebildet, mit einem innenliegenden, zylinderförmigen, rotierenden Fermenter 1 aus Kunststoff. Der Fermenter liegt in einem Heißwasserbad 2 von etwa 55 °C, das durch einen Durchlauferhitzer auf konstanter Arbeitstemperatur gehalten wird. Die in einer Vorkammer angesammelten, eingelagerten Abfälle und Reststoffe werden homogenisiert und mittels einer Fördereinrichtung in den Fermenter 1 eingebracht, wo das Substrat bei konstanter Temperatur unter ständiger Gaserzeugung ausfault.

Das ausgegorene Substrat wird mit einer Handpumpe 8 entnommen. Die Anlage ist so konstruiert, daß auch bei kontinuierlichem Betrieb immer genügend Bakterien (Impfgut) im Fermenter 1 verbleiben.

Der im Fermenter 1 erzeugte Gasdruck wird über eine Leitung 3 einem Antriebsmittel 4 zugeführt, welches den Fermenter in Rotation versetzt. Dabei dreht sich der Fermenter um im Inneren des Fermenters feststehend angeordnete Mischarme 5, wodurch eine Sinkschichten- und Schwimmdeckenbildung wirksam vermieden wird. Überschüssiges Gas wird über ein Sicherheitsventil 9 in einem extern angeordneten Gasspeicher geleitet.

## Patentansprüche

1. Um seine Längsachse drehbar gelagerter zylinderförmiger Fermenter (1) für Biogasanlagen, dadurch gekennzeichnet, daß der Fermenter (1) in Wasser oder einer anderen Flüssigkeit (2) derart schwimmend gelagert ist, daß sein Eigengewicht mit der Befüllung durch den Auftrieb weitgehend kompensiert ist.

2. Fermenter nach Anspruch 1, dadurch gekennzeichnet, daß Mittel (4) vorhanden sind, die, durch den im Fermenter (1) sich aufbauenden Gasdruck angetrieben, den Fermenter (1) in Rotation versetzen.

3. Fermenter nach Anspruch 1 oder 2, gekennzeichnet durch im Inneren des Fermenters (1) angeordnete Mischarme (5), die eine Durchmischung der Befüllung während der Rotation des Fermenters (1) bewirken.

4. Fermenter nach einem der Ansprüche 1 bis 3, dadurch gekennzeichnet, daß der Fermenter (1) in einem Wasserdurchlauftank (6) angeordnet ist, der als schwimmendes Lager und als konstanter Prozesswärmespender für den Fermenter ausgebildet ist.

## Claims

1. Cylindrical fermenter (1) mounted rotatably about its longitudinal axis for biogas installations, characterised by the fact that the fermenter (1) is floatingly supported in water or another liquid (2) in such a way that its own weight with the filling is largely counterbalanced by the buoyancy.

2. Fermenter according to Claim 1, characterised by the fact that means (4) are available which, driven by the gas pressure building up in the fermenter (1) set the fermenter (1) in rotation.

3. Fermenter according to Claim 1 or 2, characterised by mixing arms (5) arranged inside the fermenter (1) which effect a thorough mixing of the filling during the rotation of the fermenter (1).

4. Fermenter according to one of Claims 1 to 3, characterised by the fact that the fermenter (1) is arranged in a water flow tank (6) which is designed as a floating support and as a constant process heat dispenser for the fermenter.

## Revendications

1. Cuve de fermentation (1) pour installations de gaz biologique de forme cylindrique montée de manière rotative autour de son axe longitudinal, caractérisée en ce qu'elle est disposée de manière flottante dans de l'eau ou dans un autre fluide (2), de telle manière que son poids propre avec le remplissage est largement compensé par la poussée verticale.

2. Cuve de fermentation selon la revendication 1, caractérisée en ce que sont prévus des moyens (4) entraînés par la pression de gaz engendrée dans la cuve, et mettant la cuve (1) en rotation.

3. Cuve de fermentation selon la revendication 1 ou 2, caractérisée par des bras de mélange (5) disposés à l'intérieur de la cuve (1) qui réalisent un brassage du remplissage pendant la rotation de la cuve.

4. Cuve de fermentation selon l'une quelconque des revendications 1 à 3, caractérisée en ce que la cuve (1) est disposée dans une citerne à circulation d'eau (6) qui forme un logement flottant et un émetteur de chaleur de procédé constant pour la cuve de fermentation.

Vorkammer

M 1:20

0 051 082